# EUROPÄISCHE PATENTSCHRIFT

(11) **EP 0 290 642 B1**
(45) Veröffentlichungstag und Bekanntmachung des Hinweises auf die Patenterteilung: **03.11.1993**
(21) Anmeldenummer: 87106835.9
(22) Anmeldetag: 12.05.1987
(51) Int. Cl.: A61L 27/00, A61L 33/00

(54) **Verfahren zur Besiedlung einer Polymeroberfläche mit menschlichen Gefässinnenhautzellen**
Method for seeding polymeric surfaces with human endothelial cells
Procédé pour ensemencer des surfaces polymériques avec des cellules endothéliales humaines

(43) Veröffentlichungstag der Anmeldung: 17.11.1988
(73) Patentinhaber: Mittermayer, Christian, Dr. med., D-52057 Aachen (DE); Klee, Doris, Dr. rer. nat., D-52074 Aachen (DE); Richter, Horst, Dr.-Ing., D-52074 Aachen (DE)
(72) Erfinder: Mittermayer, Christian, Dr. med., D-52057 Aachen (DE); Klee, Doris, Dr. rer. nat., D-52074 Aachen (DE); Richter, Horst, Dr.-Ing., D-52074 Aachen (DE)
(74) Vertreter: Schmetz, Bruno, Dipl.-Ing.

(56) Entgegenhaltungen:
- EP-A- 0 206 025
- FR-A- 2 187 849
- FR-A- 2 440 733

## Beschreibung

### Stand der Technik:

Zahlreiche Forschungsprojekte hatten und haben das Ziel, neue Polymere für den kardiovaskulären Implantateinsatz zu entwickeln. Die wichtigste Forderung, die an blutverträgliche Polymere gestellt wird, ist die Antithrombogenität der Polymeroberfläche. Zur Verwirklichung dieser Zielsetzung werden die unterschiedlichsten Lösungswege beschrieben. Neben dem Einsatz von synthetischen Homopolymeren, wie z.B. aus Polyethylenterephtalat (1, 1a, 4, 8, 10) oder Polytetrafluorethylen (4, 9) werden verschiedene Oberflächenmodifikationen für Polymere in Blutkontakt beschrieben. Als Beispiel ist die Ausrüstung von Polymeren mit Hydrogelen zu nennen (2, 2b, 3, 7), die die Adsorption von Blutproteinen zurückdrängt. Dies soll die Thrombusbildung verhindern. Ein anderer Weg ist die Oberflächenmodifikation mit biologisch aktiven Makromolekülen. Insbesondere werden die Beschichtung von Gefäßprothesen mit Heparin (11, 12) oder Albumin (6, 6a) durchgeführt. Jedoch ist bei einem Langzeiteinsatz mit dem Verlust der biologisch aktiven Proteine zu rechnen, so daß bisher der Einsatz nur für Kurzzeitbeanspruchungen, etwa als Katheter, geeignet ist.

Trotz intensiver Forschung ist es bis heute nicht gelungen, eine synthetische Polymeroberfläche zu entwickeln, die eine Plättchenaggregation vollständig und dauerhaft verhindert. Nach wie vor werden ortsständige Thromben sowie vom Blutstrom mitgeschwemmte Mikroemboli beobachtet. Beim Einsatz von Prothesen in großen Gefäßen, wie z.B. in der Bauchschlagader, ist ein Verschluß der Prothese aufgrund des großen Durchmessers und des starken Blutstromes unwahrscheinlich; problematisch ist es mit Gefäßprothesen kleinen Durchmessers (< 4 mm). Sie werden von Blutgerinnseln nach und nach blockiert. Für die Fertigung von Prothesen kleinen Durchmessers gibt es bisher keine zufriedenstellende Lösung. Dasselbe gilt für den Langzeiteinsatz von Sonden und Sensoren, die im direkten Blutkontakt stehen.

Neben der Gefahr der Blockierung des Lumens durch Thromben können darüber hinaus synthetische Polymere, die mit dem Blutstrom in Kontakt sind, das Wachstum von Mikroorganismen, insbesondere von Bakterien, fördern. Die Folge kann sein: Nahtdehiszenzen, Rupturen der Gefäße sowie die Ausbildung einer Sepsis (Blutvergiftung). Diese Folgeerscheinungen können für den Patienten gefährlicher werden als die eigentliche Grundkrankheit, die man mit dem Implantat beseitigen oder lindern wollte.

Da eine intakte Endothelzellschicht (Gefäßinnenhautzellen) der natürlichen Gefäßinnenwand den wirksamsten Schutz gegen Blutgerinnung und Bakterienwachstum darstellen, entsteht folgerichtig das Konzept, synthetische Polymere flächendeckend mit organismuseigenen, vaskulären Endothelzellen zu besiedeln. Eine Gefäßprothese, die vor oder nach der Implantation dauerhaft mit Endothelzellen besiedelt wurde, wird sich im Langzeiteinsatz als antithrombotisch erweisen.

Schon Ende der 70er Jahre wurde von ESKIN und dem Unternehmen Union Carbide Industries (1b, 2a, 5) die Idee der Endothelzellbesiedlung auf synthetischem Trägerpolymer beschrieben. Bei diesem Verfahren findet die Besiedelung auf einem Polypropylenmikrofilamentnetz, welches sich auf einem Polyurethanrücken befindet, statt. Die beschriebene Besiedlung wurde mit bovinen Endothelzellen und mit Kälbern als Versuchstiere durchgeführt.

Andere Erfolge zur lang anhaltenden Besiedlung von künstlichen Polymeren konnten im allgemeinen bei Gefäßprothesen in Tieren, im Besonderem bei Hunden und Pavianen, erzielt werden (13, 14). Hinzuweisen ist, daß die Physiologie der Endothelzellen von Species zu Species sehr unterschiedlich ist. Menschliche Endothelzellen sind besonders schwer in vitro zu züchten. Das "Preseeding" dieser Zellart auf potentielle Gefäßimplantate in der Zuchtflasche gibt große Probleme auf. Deshalb blieb der durchschlagende Erfolg bis zum klinischen Einsatz eines endothelzellbesiedelten Polymers beim Menschen bisher aus. Die Ursache liegt in der unzureichenden Systematik bei der Entwicklung eines geeigneten Trägerpolymers und in den fehlenden Kenntnissen über Adhäsionsverhalten und Funktionalität der menschlichen Gefäßinnenhautzellen.

### Darstellung der Erfindung:

Gegenstand der in den Ansprüchen gekennzeichneten Erfindung ist eine Verfahrensentwicklung zur Besiedlung von Polymeroberflächen, vorzugsweise Oberflächen auf Basis von Polyurethan (PUR), insbesondere aus Polyether-PUR, mit humanen Gefäßinnenhautzellen, um eine dauerhafte Antithrombogenität der Oberfläche für den Implantateinsatz von Gefäßen und Herzersatzteilen zu erhalten. Darüber hinaus können dauerhaft im Blutkontakt stehende Sonden und Sensoren mit Endothelzellen besiedelt werden.
Nach der vorliegenden Erfindung wird vor der Besiedlung die Oberfläche eines Polymers durch die kovalente Bindung eines Biopolymers als Adhäsionsvermittler modifiziert. Da die Gefäßinnenhautzellen optimal nur auf einer extrazellulären Matrix (ECM) gedeihen, werden Proteinkomponenten der Extrazellulärmatrix, wie z.B. Kollagen IV oder Laminin als Adhäsionsvermittler eingesetzt.

Zur kovalenten Kopplung der Biopolymere werden auf die Polymeroberfläche geeignete funktionelle Gruppen gepfropft, die zur Bindung von endständigen Aminosäuregruppen oder Aminosäureketten des Biopolymers befähigt sind. Die wichtigste Bedingung bei der Kopplung ist der Erhalt der biologischen Aktivität des Biopolymers. Damit wird einerseits eine mechanische feste Bindung des Biopolymers erzielt und die für die Haftung der natürlichen Endothelzellen notwendigen Bindungsstellen geschont. Letztere Funktionen werden vor der Zellbesiedelung mit entsprechenden Antikörpern getestet (21).

Die Maskierung des synthetischen Trägerpolymers mit zelleigenen Komponenten führt zur naturähnlichen Adhäsion und damit zum kontinuierlichen lückenlosen Wachstum von Gefäßinnenhautzellen in einem non-thrombogenen Zellrasen auf der Polymeroberfläche. Durch dieses Verfahren wird die Zeitstandfestigkeit dieser auf der modifizierten Polymeroberfläche gewachsenen Zellen im Scherstrom des Blutes erreicht (16, 19).

Gegenstand der vorliegenden Erfindung ist die Maskierung und die Besiedlung der Oberfläche eines Polymers, vorzugsweise auf der Basis von PUR, insbesonders aus Polyether-PUR, durch folgende Verfahrensschritte:
- Modifikation der Oberfläche des Trägerpolymers durch Pfropfung mit geeigneten funktionellen Gruppen.
   Die funktionellen Gruppen befähigen das Trägerpolymer zu einer dauerhaften Bindung mit einem Biopolymer.
- Kovalente Bindung von endständigen Aminosäuregruppen oder Aminosäureketten eines Biopolymers an die funktionellen Gruppen des Trägerpolymers.
   Diese Art der Bindung gewährleistet den Erhalt der biologischen Aktivität des Biopolymers.
   Das Biopolymer ist der Adhäsionsvermittler zu den Gefäßinnenhautzellen. Als Adhäsionsvermittler werden Proteinkomponenten der Extrazellulärmatrix, wie z.B. Kollagen IV oder Laminin eingesetzt.
- Besiedlung des so mit Komponenten der Extrazellulärmatrix vorbereiteten und maskierten Trägerpolymers mit menschlichen Endothelzellen.

Die jeweiligen Schritte des Verfahrens nach der vorliegenden Erfindung zur Besiedlung einer Polymeroberfläche mit Gefäßinnenhautzellen, um eine langfristige Antithrombogenität zu erreichen, und die chemisch-biologischen Reaktionsmechanismen werden nachstehend nochmals detailliert beschrieben.

### Ausführungsbeispiel:

An die Oberfläche einer hydrolytisch und mechanisch stabilen Polyetherurethanfolie mit geringem Urethangruppengehalt werden funktionelle Gruppen, wie z.B. Carboxyl- Hydroxyl- oder Aminogruppen gepfropft.

Die Pfropfung von Carboxylgruppen an die Oberfläche eines PUR mittels Acrylsäuremonomeren durch radioaktive Bestrahlung wurde schon beschrieben (22). Im vorliegenden Beispiel erfolgt die Pfropfung von Carboxylgruppen an die Polymeroberfläche mittels Plasmapolymerisation in Gegenwart von Acrylsäuremonomeren, die eine stabile Polyacrylsäurebeschichtung bilden.

Das Verfahren der Plasmapolymerisation wird ebenfalls zur BeSchichtung des Trägerspolymers mit Polyvinylacetat unter Anwesenheit von Vinylacetatmonomeren eingesetzt.
Die verschiedenen Pfropfungen erfolgen in einer konventionellen Plasmapolymerisationsanlage. In der Anlage wird das Trägerpolymer durch Mikrowellenplasma beschichtet. Die zu beachtenden Parameter sind die Leistung des Mikrowellengenerators (Frequenz, Feldstärke), der Einspeisungsabstand (Folienoberfläche, Plasmazündung), der Partialdruck des Monomers, die Gaszusammensetzung und die Behandlungsdauer.
Die Parameter sind eine Funktion der Konfiguration der Polymerisationsanlage und der Eigenschaften des eingesetzten Monomers.

Nach der Plasmapolymerisation werden die freien Acetatgruppen mit alkoholischer Kalilauge verseift, um an der Oberfläche des Trägerpolymers freie Hydroxylgruppen zu erhalten.
Die Proteinkopplung erfolgt nach den gängigen Methoden zur Inmobilisierung von Proteinen an polymeren Trägern, siehe Houben-Wyhl, Bd. 14/1/2.

Beim Vorliegen von freien Carboxylgruppen erfolgt die direkte Proteinkopplung mit endständigen bzw. in den Seitenketten befindlichen Aminogruppen nach folgendem Schema: 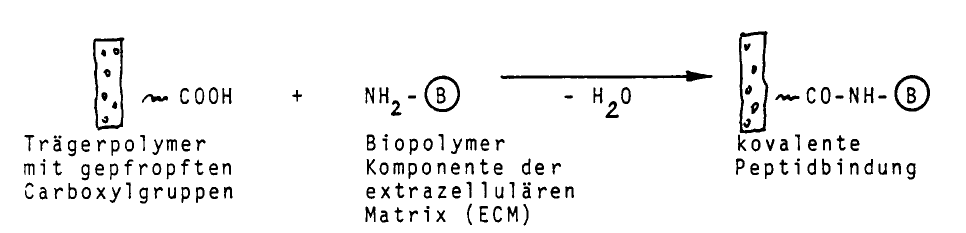
Im Falle von vorliegenden Hydroxylgruppen wird vor der Proteinkopplung in einem Zwischenschritt ein bifunktioneller Spacer, vorzugsweise Diisocyanat, eingeführt, wie unten gezeigt ist. 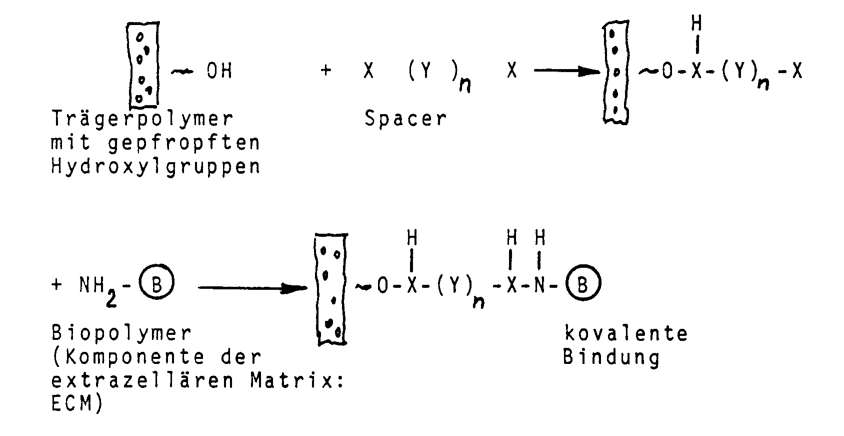
Nach Pfropfung von Aminogruppen an die Polyetherurethanoberfläche wird an endständige oder in den Seitenketten enthaltene Carboxylgruppen des Biopolymers gekoppelt, wie im folgenden Schema dargestellt ist. 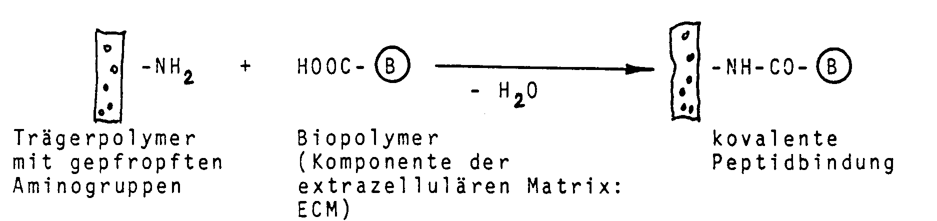
Die Auswahl des Biopolymers beschränkt sich auf die Komponenten der in Schichten aufgebauten extrazellulären Matrix (sogenannte Basalmembran), die in direktem Kontakt zu der Zelle stehen und zur Zelladhäsion führen. Im einzelnen handelt es sich um Kollagen IV und Laminin. Mit den entsprechenden Antikörpern wird der Erhalt der biologischen Aktivität des kovalent gebundenen Proteins überprüft und damit die typische Polarität dieser zellfreien Schicht gewährleistet.
Nach der Proteinkopplung erfolgt die Besiedlung des modifizierten Trägerpolymers mit humanen Gefäßinnenhautzellen aus einer semikonfluenten Kultur, bis eine konfluente lückenlose Gefäßinnenhautzellschicht erreicht ist. Die Charakterisierung menschlicher Endothelzellen erfolgt durch Nachweis von Faktor VIII related antigen (15), durch besondere Wachstumskriterien (17) sowie durch Ausbildung eines typischen Zytoskeletts nach Konfluenz (18). Die Frage der Non-Thrombogenität sowie der Zeitstandfestigkeit unter Scherbelastung werden in einem eigens konstruierten Apparat getestet (19). Sowohl frische als auch kryokonservierte Zellen (20) können zur Aussaat verwendet werden. Während der Besiedlung scheiden die Endothelzellen an der dem Blutstrom abgewandten (abluminalen) Seite eine extrazelluläre Matrix (ECM) ab (siehe Abb. 1), die die Verankerung der Zellen am Polymer zusätzlich festigen.
Insgesamt wird damit eine antithrombogene Oberfläche geschaffen, die den Eigenschaften eines natürlichen intakten Gefäßes am nächsten ist.

### Literatur:

/1/ Gebelein,C.G.; Koblitz,F.F. (Editors)
   Polymer Science and Technology, Volume 14
   "Biomedical and Dental Applications of Polymers",
   Plenum Press, New York, London (1981)
/1a/ Akutsu,T.; Yamamoto,N.; Serrato,M.A.; Denning,J.; Drummond,M.A.
   "Plastic Materials used for Fabrication of Blood Pumps", 119-142
/1b/ Eskin,S.G.; Navarro,L.T.; Sybers,H.D.; O'Bannon,W.; De Bakey,M.E.
   "Tissue Cultured Cells: Potential Blood compatible Linings for Cardiovascular Protheses", 143-162
/2/ Szycher,M.; Robinson,W.J. (Editors)
   "Synthetic Biomedical Polymers, Concepts and Applications",
   Technomic Publishing Co, Inc., Westport (1980)
/2a/ Tittmann,F.R.; Beach,W.F.
   "Parylene coated Polypropylene Microfibres as Cell Seeding Substractes", 117-132
/2b/ Ratner,B.D.; Hoffmann,A.S.
   "Surface grafted Polymers for Biomedical Applications", 133-152
/3/ Hoffmann,A.S.
   Advances in Polymer Science, 57, Polymers in Medicine,
   Springer-Verlag Berlin, Heidelberg, New York, Tokyo (1984)
   "Ionizing Radiation and Gas Plasma or Glow Discharge Treatments for preparation of Novel Polymeric Biomaterials", 141-1
/4/ Sawyer,P.N.; O'Shaughnessy,A.M.; Sopie,Z.
   Journ. of Biomed. Mat. Res., 19, 991-1010 (1985)
   "Development and performance characteristics of a new vascular graft"
/5/ Mansfield,P.B.; Wechezak,A.R.; Sauvage,L.R.
   Trans. Amer. Soc. Artif. Int. Organs, 21, 264-272 (1975)
   "Preventing Thrombus on Artificial Vascular Surfaces: true Endothelial Cell Linings"
/6/ Gesellschaft für Strahlen- und Umweltforschung mbH
   München, Bereich Projektträgerschaften
   "Materiale und Systeme zur Organunterstützung, Biokompatible Werkstoffe", Projektbericht 1982/83, BPT-Bericht 8 (1984)
/6a/ Müller,F.J.
   Vorhaben MSO 112, 23-28
   "Entwicklung antithrombogener Kunststoffoberflächen: Aufbringen dynamischer Protein-Albumin-Schichten"
/7/ Jansen,B.; Ellinghorst,G.
   Journ. of Biomed. Mat. Res., 19, 1085-1099 (1985)
   "Modification of polyetherurethane for biomedical application by radiation-induced grafting.
   I. Grafting procedure, determination of mechanical properties and chemical modification of grafted films"
/8/ Herring,M.B.; Russell,B.; Jersild,R.A.; Boxer,L.; Gardner,A.; Glover,J.
   Ann. Surg., 190, 84-90 (1979)
   "Seeding Arterial Protheses with Vascular Endothelium"
/9/ Gore,W.L.
   Science (1978)
   "Gore-Tex expanded PTFE - a new biocompatible material"
/10/ Hoffmann,A.S.
   Journ. of Appl. Poly. Sci: Appl. Poly. Symp., 31, 313-334 (1977)
   "Medical Applications of Polymeric Fibres"
/11/ Fischer,A.M.; Mauzac,M.; Tapon-Bretaudiere,J.; Jozefonvicz,J. Biomaterials, 6, 198-202 (1985)
   "Anticoagulat activity of dextran derivates Part II: Mechanism of thrombin inactivation"
/12/ Jozefowicz,M.; Jozefonvicz,J.
   Pure and Appl. Chem., 56, 1335-1344 (1984)
   "Antithrombogenic Polymers"
/13/ Shepard,A.D. et al.
   Surgery, 99, 318-325 (1986)
   "Endothelial cell ceeding of small-caliber synthetic graft in the baboon"
/14/ Herring,M.B.; Russell,D.; Jersild,R.A.; Boxer,L.; Gardner,A.; Glover,J.
   Ann. Surg., 190, 84-90 (1979)
/15/ Schöffel,U.; Kopp,K.H.; Männer,H.; Vogel,F.; Mittermayer,C.
   European Journal of Clinical Investigation, 12, 165-171 (1982)
   "Human endothelial cell proliferation inhibiting activity in the sera of patients suffering from 'shock' or 'sepsis'"
/16/ Levesque,M.J.; Nerem,R.M.
   Journ. of Biomed. Eng., 107, 341-347 (1985)
   "The Elongation and Orientation of Cultured Endothelial Cells in Response to Shear Stress"
/17/ Schöffel,U.; Männer,H.; Shiga, J.; Mittermayer,C. Path. Res. Pract., 175, 348-364 (1982)
   "Proliferative Response of Human Endothelial Cultures to Various Types and Treatments of Human Sera to Culture Treatments and to Various Oxygen Concentrations"
/18/ Schnittler,H.J.; Franke,R.P.; Fuhrmann,R.; Mittermayer,C.; Drenckhahn,D.
   34th ETCS-Meeting, Heidelberg
   Europ. Journ. Cell Biol., 42 (Suppl. 15), 18, ES 3 (1986)
   "Development of Stressfibres (SF) and Micrfilaments in Human Umbilical Venous Endothelial Cells (HUVEC) in Dependence of Varying Culture Periods within one Passage"
/19/ Franke,R.P.; Gräfe,M.; Schnittler,H.J.; Seiffge,D.; Drenckhahn,D.; Mittermayer,C.
   Nature, 307, 648-649 (1984)
   "Induction of human vascular endothelial stress fibres by fluid shear stress"
/20/ Schäfer,A.T.; Körber,C.; Scheiwe,M.W.; Rau,G.; Franke,R.P.; Mittermayer,C.
   Cryo-Letters, 7, 55-67 (1986)
   "Preliminary investigation of osmotic properties and freezing behavior of human endothelial cells"
/21/ Cheresh,D.A.; Pierschbacher,M.D.; Herzig,M.A.; Mujoo,K.
   Journ. of Cell Biol., 102, 688-696 (1986)
   "Disialogangliosides GD2 and GD3 Are Involved in the Attachment of Human Melanoma and Neuroblastoma Cells to Extracellular Matrix Proteins"
/22/ DEMANDE DE BREVENT D INVENTION,
   No. 79 27476 (Fr - A - 2440 733)
   "Dispositif chirurgical biologiquement compatible, comprenant une matiere plastique"
   22/FYDELOR, P.J., TAYLOR, D.E.M.

## Patentansprüche

1. Verfahren zur Besiedlung einer künstlichen Polymeroberfläche mit humanen Gefäßinnenhautzellen, um eine dauerhafte Antithrombogenität zu erreichen durch die hohe Adhäsion von humanen Gefäßinnenhautzellen, die als Monolayer auf der nach Verfahren 1a - 1b modifizierten Polymeroberfläche vor dem Blutkontakt angesiedelt werden, wobei
a) An eine Polymeroberfläche, vorzugsweise eine Oberfläche auf der Basis von PUR, insbesondere aus Polyether-Polyurethan, Hydroxylgruppen an die Oberfläche gepfropft werden mittels Plasmapolymerisation mit Vinylacetat und anschließender Verseifung;
b) um über einen zwischengeschalteten bifunktionellen Spacer mit einer der zahlreichen Proteinkomponenten der extrazellulären Matrix (ECM), vorzugsweise mit Kollagen IV oder Laminin, gekoppelt zu werden.

2. Verfahren nach Anspruch 1 dadurch gekennzeichnet, daß beim Schritt a. Aminogruppen an die Oberfläche gepfropft werden und die Proteinkopplung an endständige oder in den Seitenketten enthaltene Carboxylgruppen des zu koppelnden Proteins erfolgt.

3. Verfahren nach einem der vorstehenden Ansprüche dadurch gekennzeichnet, daß den beschriebenen Schritten a. und b. die Besiedlung von humanen Gefäßinnenhautzellen folgt.

## Claims

1. A method of colonising a synthetic polymer surface with human vessel endothelium cells so as to achieve a long-lasting antithrombogeneity as a result of the high degree of adhesion of human vessel endothelium cells which are colonised as a monolayer on the polymer surface modified in accordance with method 1a - 1b prior to blood contact, wherein
a) on a polymer surface, preferably a surface based on polyurethane (PUR), in particular of polyether polyurethane, hydroxyl groups are grafted on to the surface by means of plasma-polymerisation with vinyl acetate and subsequent saponification;
b) so as to be coupled via an interposed bifunctional spacer with one of the numerous protein components of the extracellular matrix (ECM), preferably with collagen IV or laminin.

2. A method according to Claim 1, characterised in that in step a) amino groups are grafted on to the surface and the protein coupling is effected to carboxyl groups being present at the end chain or contained in the side chains of the protein to be coupled.

3. A method according to either one of the preceding Claims, characterised in that the above-described steps a) and b) are followed by the colonisation of human vessel endothelium cells.

## Revendications

1. Procédé d'ensemencement d'une surface polymère artificielle à l'aide de cellules endothéliales humaines afin d'atteindre une antithrombogénéité durable grâce à l'adhérence élevée de cellules endothéliales humaines qui sont ensemencées sous forme de monocouche sur la surface polymère modifiée selon le procédé 1a-1b avant le contact avec le sang, dans lequel
a) sur une surface polymère, de préférence une surface à base de polyuréthanne ( PUR), en particulier de polyéther-polyuréthanne, des groupes hydroxyles sont greffés à la surface au moyen d'une polymérisation au plasma avec de l'acétate de vinyle, suivie d'une saponification;
b) afin d'être couplés, par l'intermédiaire d'un spacer bifonctionnel intercalé, avec une des nombreuses composantes protéiniques de la matrice extracellulaire (ECM), de préférence avec du collagène IV ou de la laminine.

2. Procédé selon la revendication 1 caractérisé en ce que, à l'étape a), des groupes amino sont greffés sur la surface et le couplage protéinique est réalisé sur des groupes carboxyles, situés en fin de chaîne ou contenus dans des chaînes latérales, de la protéine à accoupler.

3. Procédé selon l'une quelconque des revendications précédentes, caractérisé en ce que l'ensemencement de cellules endothéliales humaines suit les étapes a) et b) décrites.
